# EUROPEAN PATENT SPECIFICATION

(11) **EP 3 700 451 B1**
(45) Date of publication and mention of the grant of the patent: **03.08.2022**
(21) Application number: 18801137.3
(22) Date of filing: 23.10.2018
(51) Int. Cl.: A61B 18/14, A61N 1/05, A61N 1/32, A61N 1/36

(54) **VOLUME-FILLING LEADS FOR TREATMENT OF CANCER WITH ELECTRIC FIELDS**
VOLUMENFÜLLUNGSELEKTRODEN ZUR BEHANDLUNG VON KREBS MIT ELEKTRISCHEN FELDERN
FILS DE REMPLISSAGE DE VOLUME POUR LE TRAITEMENT DU CANCER À L'AIDE DE CHAMPS ÉLECTRIQUES

(30) Priority: 23.10.2017 US 201762575693 P; 22.10.2018 US 201816167087
(43) Date of publication of application: 02.09.2020
(73) Proprietor: Cardiac Pacemakers, Inc., St. Paul, Minnesota 55112 (US)
(72) Inventor: SCHMIDT, Brian L., White Bear Lake, Minnesota 55110 (US); LUDWIG, Jacob M., Isanti, Minnesota 55040 (US); HAASL, Benjamin J., Forest Lake, Minnesota 55025 (US); KANE, Michael J., St. Paul, Minnesota 55105 (US)
(74) Representative: Vossius & Partner Patentanwälte Rechtsanwälte mbB
(86) International application number: PCT/US2018/057120
(87) International publication number: WO 2019/084016

(56) References cited:
- US-A1- 2005 004 507
- US-A1- 2005 288 730
- US-A1- 2010 298 895
- US-A1- 2013 165 916
- US-A1- 2017 105 793

## Description

### Field

Embodiments herein relate to medical devices including volume filling leads to treat cancerous tumors within a bodily tissue. More specifically, embodiments herein relate to using volume filling leads and electrodes configured to generate therapeutic electric fields at the site of a cancerous tumor.

### Background

According to the American Cancer Society, cancer accounts for nearly 25% of the deaths that occur in the United States each year. The current standard of care for cancerous tumors can include first-line therapies such as surgery, radiation therapy, and chemotherapy. Additional second-line therapies can include radioactive seeding, cryotherapy, hormone or biologics therapy, ablation, and the like. Combinations of first-line therapies and second-line therapies can also be a benefit to patients if one particular therapy on its own is not effective.

Cancerous tumors can form if one normal cell in any part of the body mutates and then begins to grow and multiply too much and too quickly. Cancerous tumors can be a result of a genetic mutation to the cellular DNA or RNA that arises during cell division, an external stimulus such as ionizing or non-ionizing radiation, exposure to a carcinogen, or a result of a hereditary gene mutation. Regardless of the etiology, many cancerous tumors are the result of unchecked rapid cellular division.

Mitosis is the process of cellular division that is a part of the cell cycle for all somatic cells in the body, including many types of cancerous cells. Mitosis includes four basic phases: prophase, metaphase, anaphase, and telophase. Just prior to prophase, a cell will copy its chromosomes to create two identical sister chromatids. During prophase, the chromosomes start to condense and the nuclear membrane surrounding the nucleus disappears. The mitotic spindle also begins to form during prophase. The mitotic spindle includes a self-organized bipolar array of microtubules and centrosomes. Microtubules are generally formed from the polymerization of the highly polar alpha-tubulin and beta-tubulin proteins. Centrosomes are similarly protein-based organelles, two of which migrate to opposite sides of the dividing cell at this phase. The negatively charged end of the microtubules attach to the centrosomes. The positively charged end of the microtubules radiate toward the equator of the dividing cell where they eventually attach to a kinetochore of each sister chromatid. Metaphase can be defined by all chromosomes being aligned at the equator of the dividing cell and bound in the mitotic spindle. An equal number of sister chromatids are then pulled toward opposite ends of the cell during anaphase. Once all chromosomes have been separated, the process of telophase begins, where the cell membrane begins to form a cleavage furrow between the two newly forming sister cells, and cell division becomes complete once the cells physically separate from one another in a process called cytokinesis. The related prior art is disclosed by US2005288730.

### Summary

Embodiments herein relate to medical devices including volume filling leads to treat cancerous tumors within a bodily tissue. In a first aspect, a lead for a cancer treatment system is provided. The lead can include a lead body having a proximal end and a distal end, where the lead body can define a lumen. The lead can also include an expandable lead head connected to the distal end of the lead body. The lead head can be configured to be expanded between a first non-expanded position and a second expanded position in order to fill an intracorporeal void. The lead can also include two or more electrodes disposed on an outer surface of the lead head and two or more electrical conductors configured to provide electrical communication between the two or more electrodes and the proximal end of the lead body, wherein the expandable lead head comprises an expandable balloon configured to assume an amorphous shape when the expandable balloon is expanded into a second expanded position.

In a second aspect, in addition to one or more of the preceding or following aspects, or in the alternative to some aspects, the lead head can include a proximal end and a distal end, the lead head including one or more flexible supports extending between the proximal end and the distal end.

In a third aspect, in addition to one or more of the preceding or following aspects, or in the alternative to some aspects, the one or more flexible supports can be biased to flex outward causing the lead head to assume the second expanded position.

In a fourth aspect, in addition to one or more of the preceding or following aspects, or in the alternative to some aspects, the one or more flexible supports comprising a proximal end and a distal end, wherein at least one of the proximal end and the distal end of the flexible supports are configured to move relative to the lead body causing the flexible supports to flex outward.

In a fifth aspect, in addition to one or more of the preceding or following aspects, or in the alternative to some aspects, the diameter of the expandable lead head is less than 2 centimeters in the first non-expanded position and greater than 2 centimeters in the second expanded position.

In a sixth aspect, in addition to one or more of the preceding or following aspects, or in the alternative to some aspects, the two or more electrodes disposed on the one or more flexible supports.

In a seventh aspect, in addition to one or more of the preceding or following aspects, or in the alternative to some aspects, the lead head include an expandable balloon.

In an eighth aspect, in addition to one or more of the preceding or following aspects, or in the alternative to some aspects, where two or more electrodes are disposed outside of the expandable balloon.

In a fifteenth aspect, in addition to one or more of the preceding or following aspects, or in the alternative to some aspects, a lead for a cancer treatment system is provided. The lead can include a lead body having a proximal end and a distal end, where the lead body can define a lumen. The lead can include two or more electrodes disposed on an outer surface of the distal end of the lead body and two or more electrical conductors configured to provide electrical communication between the two or more electrodes and the proximal end of the lead body. The distal end of the lead body can include a helix.

In a sixteenth aspect, in addition to one or more of the preceding or following aspects, or in the alternative to some aspects, the one or more electrodes are disposed on surfaces of the helix facing outward from a central axis of the helix.

In a seventeenth aspect, in addition to one or more of the preceding or following aspects, or in the alternative to some aspects, the helix is flexible and can decrease in diameter as pressure applied inward is increased and can increase in diameter as pressure applied inward is decreased.

In an eighteenth aspect, in addition to one or more of the preceding or following aspects, or in the alternative to some aspects, a helix can have an outer diameter of at least 1 centimeter when no pressure is applied inward.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### Brief Description of the Figures

Aspects may be more completely understood in connection with the following drawings, in which:
FIG. 1 is a schematic view of a medical system in accordance with various embodiments herein.
FIG. 2 is a schematic view of a medical system in accordance with various embodiments herein.
FIG. 3 is a schematic cross-sectional view of a medical device in accordance with various embodiments herein.
FIG. 4 is a schematic view of a medical device in accordance with various embodiments herein.
FIG. 5 is a schematic diagram of components of a medical device in accordance with various embodiments herein.
FIG. 6 is a schematic view of a medical device in accordance with various embodiments herein.
FIG. 7 is a schematic view of a lead in accordance with various embodiments herein.
FIG. 8 is a schematic view of a lead in accordance with various embodiments herein.
FIG. 9 is a schematic view of a lead in accordance with various embodiments herein.
FIG. 10 is a schematic view of a lead in accordance with various embodiments herein.
FIG. 11 is a schematic view of a lead in accordance with various embodiments herein.
FIG. 12 is a schematic view of a lead in accordance with various embodiments herein.
FIG. 13 is a schematic diagram of a lead in accordance with various embodiments herein.
FIG. 14 is a schematic cross-sectional view of a lead is shown along line 14-14' of FIG. 13.
FIG. 15 is a plot of an exemplary therapy parameter in accordance with various embodiments herein.

### Detailed Description

As referenced above, many cancerous tumors can result from unchecked rapid cellular division. Some traditional first-line therapies to treat cancerous tumors can include surgery, radiation therapy, and chemotherapy. However, many first-line therapies have undesirable concomitant side effects, such as fatigue, hair loss, immunosuppression, and long surgical recovery times, to name a few.

While not intending to be bound by theory, it is believed that alternating electric fields can disrupt mitosis within a cancerous tumor by interfering with the dipole alignment of key proteins involved in cellular division; tubulin and septin in particular. The polymerization of tubulin proteins that form microtubule spindle fibers can be disrupted, thus preventing the formation of spindle fibers required for chromosome separation. This can halt cellular division at the metaphase stage of mitosis. In some instances, an alternating electric field can halt polymerization of already growing spindle fibers, leading to incomplete spindles and unequal chromosome separation during anaphase, should the cell survive that long. In each case, halting microtubule spindle formation and unequal chromosome separation during anaphase caused by incomplete polymerization of microtubules can result in apoptosis (i.e., programmed cell death).

It is also believed that alternating electric fields can lead to increased electric field density near the cleavage furrow of the dividing cells during telophase. An increased electric field density in the region of the cleavage furrow can result in dielectrophoresis of charged macromolecules, such as proteins and nucleic acids, toward the high electric field density at the furrow. The unequal concentration of key macromolecules required for cellular division at the site of the cleavage furrow can disrupt the final separation of the sister cells during telophase and eventually lead to apoptosis.

The shape and size of an electric field can be modulated by the positioning of electrodes in space and by varying the electric field at a number of different electrode configurations. Sometimes, the shape of an electric field can be manipulated by alternating or switching the polarity of discrete electrodes within an individual array of electrodes or within the entire medical device system.

When a cancerous tumor has been surgically resected, often times a void remains. Treatment of the void with a volume-filling electric field can be performed to provide an additional line of treatment against any cancerous cells that may remain at the surgical margins following resection.

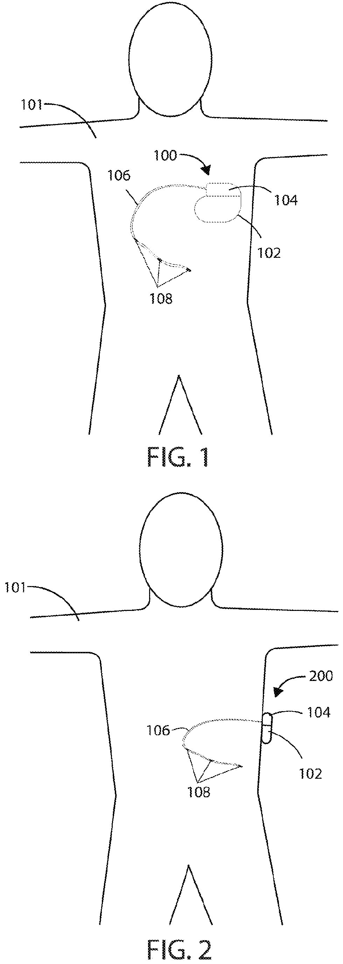

Referring now to FIG. 1, a schematic view is shown of a medical device 100 in accordance with various embodiments herein. The medical device 100 can be implanted entirely within the body of a patient 101 at or near the site of a cancerous tumor located within a bodily tissue. Various implant sites can be used including areas such as in the limbs, the upper torso, the abdominal area, the head, and the like.

Referring now to FIG. 2, another schematic view is shown of a medical device 200 in accordance with various embodiments herein. The medical device 200 can be partially implanted within the body of a patient 101. In some embodiments, the medical device can be partially implanted and partially external to the body of a patient. In other embodiments, a partially implanted medical device can include a transcutaneous connection between components disposed internal to the body and external to the body. A partially implanted medical device can wirelessly communicate with a partially external portion of a medical device over a wireless connection.

In some embodiments, a portion of the medical device can be entirely implanted and a portion of the medical device can be entirely external. For example, in some embodiments, one or more electrodes or leads can be entirely implanted within the body, whereas the portion of the medical device that generates an electric field, such as an electric field generator, can be entirely external to the body. It will be appreciated that in some embodiments described herein, the electric field generators described can include the many of the same components as and can be configured to perform many of the same functions as a pulse generator. In embodiments where a portion of a medical device is entirely implanted and a portion of the medical device is entirely external, the portion of the medical device that is entirely external can communicate wirelessly with the portion of the medical device that is entirely internal. However, in other embodiments a wired connection can be used.

The medical device 100 or medical device 200 can include a housing 102 and a header 104 coupled to the housing 102. Various materials can be used. However, in some embodiments, the housing 102 can be formed of a material such as a metal, ceramic, polymer, composite, or the like. In some embodiments, the housing 102, or one or more portions thereof, can be formed of titanium. The header 104 can be formed of various materials, but in some embodiments the header 104 can be formed of a translucent polymer such as an epoxy material. In some embodiments the header 104 can be hollow. In other embodiments the header 104 can be filled with components and/or structural materials such as epoxy or another material such that it is non-hollow.

In some embodiments where a portion of the medical device 100 or 200 is partially external, the header 104 and housing 102 can be surrounded by a protective casing made of durable polymeric material. In other embodiments, where a portion of the medical device 100 or 200 is partially external, the header 104 and housing 102 can be surrounded by a protective casing made of a combination of polymeric material, metallic material, and/or glass material.

The header 104 can be coupled to one or more leads 106. The header 104 can serve to provide fixation of the proximal end of one or more leads 106 and electrically couple the one or more leads 106 to one or more components within the housing 102. The one or more leads 106 can include one or more electrodes 108 disposed along the length of the electrical leads 106. In some embodiments, electrodes 108 can include electric field generating electrodes and in other embodiments electrodes 108 can include electric field sensing electrodes. In some embodiments, leads 106 can include both electric field generating and electric field sensing electrodes. In other embodiments, leads 106 can include any number of electrodes that are both electric field sensing and electric field generating. It will be appreciated that while many embodiments of medical devices herein are designed to function with leads, leadless medical devices that generate electrical fields are also contemplated herein.

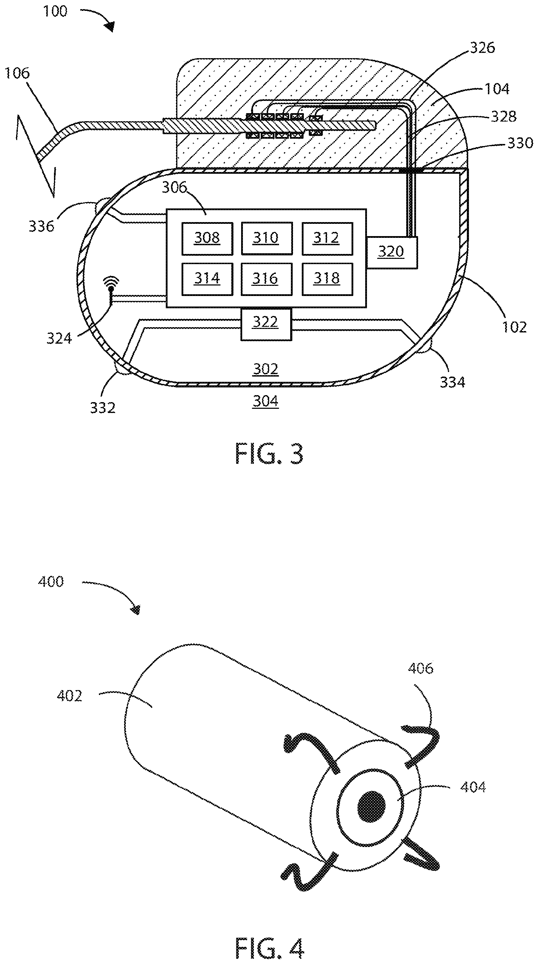

Referring now to FIG. 3, a schematic cross-sectional view of medical device 100 is shown in accordance with various embodiments herein. Housing 102 can define an interior volume 302 that can be hollow and that in some embodiments is hermetically sealed off from the area 304 outside of medical device 100. In other embodiments the housing 102 can be filled with components and/or structural materials such that it is non-hollow. The medical device 100 can include control circuitry 306, which can include various components 308, 310, 312, 314, 316, and 318 disposed within housing 102. In some embodiments, these components can be integrated and in other embodiments these components can be separate. In yet other embodiments, there can be a combination of both integrated and separate components. The medical device 100 can also include an antenna 324, to allow for unidirectional or bidirectional wireless data communication. In some embodiments, the components of medical device 100 can include an inductive energy receiver coil (not shown) communicatively coupled or attached thereto to facilitate transcutaneous recharging of the medical device via recharging circuitry.

The various components 308, 310, 312, 314, 316, and 318 of control circuitry 306 can include, but are not limited to, a microprocessor, memory circuit (such as random access memory (RAM) and/or read only memory (ROM)), recorder circuitry, controller circuit, a telemetry circuit, a power supply circuit (such as a battery), a timing circuit, and an application specific integrated circuit (ASIC), a recharging circuit, amongst others. Control circuitry 306 can be in communication with an electric field generating circuit 320 that can be configured to generate electric current to create one or more fields. The electric field generating circuit 320 can be integrated with the control circuitry 306 or can be a separate component from control circuitry 306. Control circuitry 306 can be configured to control delivery of electric current from the electric field generating circuit 320. In some embodiments, the electric field generating circuit 320 can be present in a portion of the medical device that is external to the body.

In some embodiments, the control circuitry 306 can be configured to direct the electric field generating circuit 320 to deliver an electric field using one or more frequencies selected from a range of between 10 kHz to 1 MHz. In some embodiments, the control circuitry 306 can be configured to direct the electric field generating circuit 320 to deliver an electric field at one or more frequencies selected from a range of between 100 kHz to 500 kHz. In some embodiments, the control circuitry 306 can be configured to direct the electric field generating circuit 320 to deliver an electric field at one or more frequencies selected from a range of between 100 kHz to 300 kHz. In some embodiments, the control circuitry 306 can be configured to direct the electric field generating circuit 320 to periodically deliver an electric field using one or more frequencies greater than 1 MHz.

In some embodiments, the electric field can be effective in disrupting cellular mitosis in cancerous cells. The electric field can be delivered to the site of a cancerous tumor along more than one vector. In some examples, the electric field can be delivered along at least one vector, including at least one of the lead electrodes. In some embodiments, at least two vectors with spatial diversity between the two vectors can be used. The vectors can be spatially separated (e.g., the vectors can be disposed at an angle with respect to one another) by at least about 10, 20, 30, 40, 50, 60, 70, 80 or 90 degrees.

A desired electric field strength can be achieved by delivering an electric current between two electrodes. The specific current and voltage at which the electric field is delivered can vary and can be adjusted to achieve the desired electric field strength at the site of the tissue to be treated. In some embodiments, the control circuitry 306 can be configured to direct the electric field generating circuit 320 to deliver an electric field using currents ranging from 1 mAmp to 1000 mAmp to the site of a cancerous tumor. In some embodiments, the control circuitry 306 can be configured to direct the electric field generating circuit 320 to deliver an electric field using currents ranging from 20 mAmp to 500 mAmp to the site of a cancerous tumor. In some embodiments, the control circuitry 306 can be configured to direct the electric field generating circuit 320 to deliver an electric field using currents ranging from 30 mAmp to 300 mAmp to the site of a cancerous tumor.

In some embodiments, the control circuitry 306 can be configured to direct the electric field generating circuit 320 to deliver an electric field using currents including 1 mAmp, 2 mAmp, 3 mAmp, 4 mAmp, 5 mAmp, 6 mAmp, 7 mAmp, 8 mAmp, 9 mAmp, 10 mAmp, 15 mAmp, 20 mAmp, 25 mAmp, 30 mAmp, 35 mAmp, 40 mAmp, 45 mAmp, 50 mAmp, 60 mAmp, 70 mAmp, 80 mAmp, 90 mAmp, 100 mAmp, 125 mAmp, 150 mAmp, 175 mAmp , 200 mAmp, 225 mAmp, 250 mAmp, 275 mAmp, 300 mAmp, 325 mAmp, 350 mAmp, 375 mAmp, 400 mAmp, 425 mAmp, 450 mAmp, 475 mAmp, 500 mAmp, 525 mAmp, 550 mAmp, 575 mAmp, 600 mAmp, 625 mAmp, 650 mAmp, 675 mAmp, 700 mAmp, 725 mAmp, 750 mAmp, 775 mAmp, 800 mAmp, 825 mAmp, 850 mAmp, 875 mAmp, 900 mAmp, 925 mAmp, 950 mAmp, 975 mAmp, or 1000 mAmp. It will be appreciated that the control circuitry can be configured to direct the electric field generating circuit 320 to deliver an electric field at a current falling within a range, wherein any of the forgoing currents can serve as the lower or upper bound of the range, provided that the lower bound of the range is a value less than the upper bound of the range.

In some embodiments, the control circuitry 306 can be configured to direct the electric field generating circuit 320 to deliver an electric field using voltages ranging from 1 Vᵣₘₛ to 50 Vᵣₘₛ to the site of a cancerous tumor. In some embodiments, the control circuitry 306 can be configured to direct the electric field generating circuit 320 to deliver an electric field using voltages ranging from 5 Vᵣₘₛ to 30 Vᵣₘₛ to the site of a cancerous tumor. In some embodiments, the control circuitry 306 can be configured to direct the electric field generating circuit 320 to deliver an electric field using voltages ranging from 10 Vᵣₘₛ to 20 Vᵣₘₛ to the site of a cancerous tumor.

In some embodiments, the control circuitry 306 can be configured to direct the electric field generating circuit 320 to deliver an electric field using one or more voltages including 1 Vᵣₘₛ, 2 Vᵣₘₛ, 3 Vᵣₘₛ, 4 Vᵣₘₛ, 5 Vᵣₘₛ, 6 Vᵣₘₛ, 7 Vᵣₘₛ, 8 Vᵣₘₛ, 9 Vᵣₘₛ, 10 Vᵣₘₛ, 15 Vᵣₘₛ, 20 Vᵣₘₛ, 25 Vᵣₘₛ, 30 Vᵣₘₛ, 35 Vᵣₘₛ, 40 Vᵣₘₛ, 45 Vᵣₘₛ, or 50 Vᵣₘₛ. It will be appreciated that the control circuitry can be configured to direct the electric field generating circuit 320 to deliver an electric field using a voltage falling within a range, wherein any of the forgoing voltages can serve as the lower or upper bound of the range, provided that the lower bound of the range is a value less than the upper bound of the range.

In some embodiments, the control circuitry 306 can be configured to direct the electric field generating circuit 320 to deliver and electric field using one or more frequencies including 10 kHz, 20 kHz, 30 kHz, 40 kHz, 50 kHz, 60 kHz, 70 kHz, 80 kHz, 90 kHz, 100 kHz, 125 kHz, 150 kHz, 175 kHz, 200 kHz, 225 kHz, 250 kHz, 275 kHz, 300 kHz, 325 kHz, 350 kHz, 375 kHz, 400 kHz, 425 kHz, 450 kHz, 475 kHz, 500 kHz, 525 kHz, 550 kHz, 575 kHz, 600 kHz, 625 kHz, 650 kHz, 675 kHz, 700 kHz, 725 kHz, 750 kHz, 775 kHz, 800 kHz, 825 kHz, 850 kHz, 875 kHz, 900 kHz, 925 kHz, 950 kHz, 975 kHz, 1 MHz. It will be appreciated that the electric field generating circuit 320 can deliver an electric field using a frequency falling within a range, wherein any of the foregoing frequencies can serve as the upper or lower bound of the range, provided that the upper bound is greater than the lower bound.

In some embodiments, the control circuitry 306 can be configured to direct the electric field generating circuit 320 to generate one or more applied electric field strengths selected from a range of between 0.25 V/cm to 1000 V/cm. In some embodiments, the control circuitry 306 can be configured to direct the electric field generating circuit 320 to generate one or more applied electric field strengths of greater than 3 V/cm. In some embodiments, the control circuitry 306 can be configured to direct the electric field generating circuit 320 to generate one or more applied electric field strengths selected from a range of between 1 V/cm to 10 V/cm. In some embodiments, the control circuitry 306 can be configured to direct the electric field generating circuit 320 to generate one or more applied electric field strengths selected from a range of between 3 V/cm to 5 V/cm.

In other embodiments, the control circuitry 306 can be configured to direct the electric field generating circuit 320 to generate one or more applied electric field strengths including 0.25 V/cm, 0.5 V/cm, 0.75 V/cm, 1.0 V/cm, 2.0 V/cm, 3.0 V/cm, 5.0 V/cm, 6.0 V/cm, 7.0 V/cm, 8.0 V/cm, 9.0 V/cm, 10.0 V/cm, 20.0 V/cm, 30.0 V/cm, 40.0 V/cm, 50.0 V/cm, 60.0 V/cm, 70.0 V/cm, 80.0 V/cm, 90.0 V/cm, 100.0 V/cm, 125.0 V/cm, 150.0 V/cm, 175.0 V/cm, 200.0 V/cm, 225.0 V/cm, 250.0 V/cm, 275.0 V/cm, 300.0 V/cm, 325.0 V/cm, 350.0 V/cm, 375.0 V/cm, 400.0 V/cm, 425.0 V/cm, 450.0 V/cm, 475.0 V/cm, 500.0 V/cm, 600.0 V/cm, 700.0 V/cm, 800.0 V/cm, 900.0 V/cm, 1000.0 V/cm. It will be appreciated that the electric field generating circuit 320 can generate an electric field having a field strength at a treatment site falling within a range, wherein any of the foregoing field strengths can serve as the upper or lower bound of the range, provided that the upper bound is greater than the lower bound.

In some embodiments, the control circuitry 306 can be configured to direct the electric field generating circuit 320 to deliver an electric field via leads 106 to the site of a cancerous tumor located within a bodily tissue. In other embodiments, the control circuitry 306 can be configured to direct the electric field generating circuit 320 to deliver an electric field via the housing 102 of medical device 100 to the site of a cancerous tumor located within a bodily tissue. In other embodiments, the control circuitry 306 can be configured to direct the electric field generating circuit 320 to deliver an electric field between leads 106 and the housing 102 of medical device 100. In some embodiments, one or more leads 106 can be in electrical communication with the electric field generating circuit 320. In some embodiments, the one or more leads 106 can include one or more electrodes 108 disposed along the length of the leads 106, where the electrodes 108 can be in electrical communication with the electric field generating circuit 320.

In some embodiments, various components within medical device 100 can include an electric field sensing circuit 322 configured to generate a signal corresponding to sensed electric fields. Electric field sensing circuit 322 can be integrated with control circuitry 306 or it can be separate from control circuitry 306.

Sensing electrodes can be disposed on or adjacent to the housing of the medical device, on one or more leads connected to the housing, on a separate device implanted near or in the tumor, or any combination of these locations. In some embodiments, the electric field sensing circuit 322 can include a first sensing electrode 332 and a second sensing electrode 334. In other embodiments, the housing 102 itself can serve as a sensing electrode for the electric field sensing circuit 322. The electrodes 332 and 334 can be in communication with the electric field sensing circuit 322. The electric field sensing circuit 322 can measure the electrical potential difference (voltage) between the first electrode 332 and the second electrode 334. In some embodiments, the electric field sensing circuit 322 can measure the electrical potential difference (voltage) between the first electrode 332 or second electrode 334, and an electrode disposed along the length of one or more leads 106. In some embodiments, the electric field sensing circuit can be configured to measure sensed electric fields and to record electric field strength in V/cm.

It will be appreciated that the electric field sensing circuit 322 can additionally measure an electrical potential difference between the first electrode 332 or the second electrode 334 and the housing 102 itself. In other embodiments, the medical device can include a third electrode 336, which can be an electric field sensing electrode or an electric field generating electrode. In some embodiments, one or more sensing electrodes can be disposed along lead 106 and can serve as additional locations for sensing an electric field. Many combinations can be imagined for measuring electrical potential difference between electrodes disposed along the length of one or more leads 106 and the housing 102 in accordance with the embodiments herein.

In some embodiments, the one or more leads 106 can be in electrical communication with the electric field generating circuit 320. The one or more leads 106 can include one or more electrodes 108, as shown in FIGS. 1 and 2. In some embodiments, various electrical conductors, such as electrical conductors 326 and 328, can pass from the header 104 through a feed-through structure 330 and into the interior volume 302 of medical device 100. As such, the electrical conductors 326 and 328 can serve to provide electrical communication between the one or more leads 106 and control circuitry 306 disposed within the interior volume 302 of the housing 102.

In some embodiments, recorder circuitry can be configured to record the data produced by the electric field sensing circuit 322 and record time stamps regarding the same. In some embodiments, the control circuitry 306 can be hardwired to execute various functions, while in other embodiments the control circuitry 306 can be directed to implement instructions executing on a microprocessor or other external computation device. A telemetry circuit can also be provided for communicating with external computation devices such as a programmer, a home-based unit, and/or a mobile unit (e.g. a cellular phone, personal computer, smart phone, tablet computer, and the like).

Referring now to FIG. 4, leadless medical device 400 is shown in accordance with the embodiments herein. The leadless medical device 400 can include a housing 402 and a header 404 coupled to the housing 402. Various materials can be used. However, in some embodiments, the housing 402 can be formed of a material such as a metal, ceramic, polymer, composite, or the like. In some embodiments, the housing 402, or one or more portions thereof, can be formed of titanium. The header 404 can be formed of various materials, but in some embodiments the header 404 can be formed of a translucent polymer such as an epoxy material. In some embodiments the header 404 can be hollow. In other embodiments the header 404 can be filled with components and/or structural materials such as epoxy or another material such that it is non-hollow. In some embodiments, leadless medical device 400 can include fixation elements 406 to keep a leadless medical device 400 positioned at or near the site of a cancerous tumor within the body. In some embodiments, fixation elements 406 can include talons, tines, helices, bias, and the like.

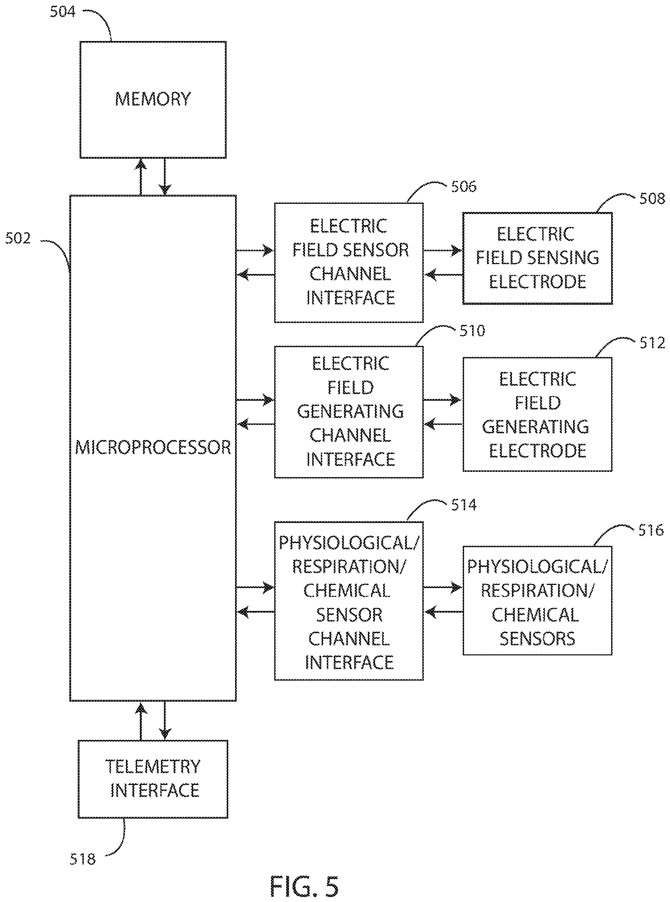

Elements of various embodiments of the medical devices described herein are shown in FIG. 5. However, it will be appreciated that some embodiments can include additional elements beyond those shown in FIG. 5. In addition, some embodiments may lack some elements shown in FIG. 5. The medical devices as embodied herein can gather information through one or more sensing channels and can output information through one or more field generating channels. A microprocessor 502 can communicate with a memory 504 via a bidirectional data bus. The memory 504 can include read only memory (ROM) or random access memory (RAM) for program storage and RAM for data storage. The microprocessor 502 can also be connected to a telemetry interface 518 for communicating with external devices such as a programmer, a home-based unit and/or a mobile unit (e.g. a cellular phone, personal computer, smart phone, tablet computer, and the like) or directly to the cloud or another communication network as facilitated by a cellular or other data communication network. In some embodiments, the medical device can include an inductive energy receiver coil interface (not shown) communicatively coupled or attached thereto to facilitate transcutaneous recharging of the medical device.

The medical device can include one or more electric field sensing electrodes 508 and one or more electric field sensor channel interfaces 506 that can communicate with a port of microprocessor 502. The medical device can also include one or more electric field generating electrodes 512 and one or more electric field generating channel interfaces 510 that can communicate with a port of microprocessor 502. The medical device can also include one or more physiological sensors, respiration sensors, or chemical sensors 516 and one or more physiological/respiration/chemical sensor channel interfaces 514 that can communicate with a port of microprocessor 502. The channel interfaces 506, 510, and 514 can include various components such as analog-to-digital converters for digitizing signal inputs, sensing amplifiers, registers which can be written to by the control circuitry in order to adjust the gain and threshold values for the sensing amplifiers, source drivers, modulators, demodulators, multiplexers, and the like.

In some embodiments, the physiological sensors can include sensors that monitor temperature, blood flow, blood pressure, and the like. In some embodiments, the respiration sensors can include sensors that monitor respiration rate, respiration peak amplitude, and the like. In some embodiments, the chemical sensors can measure the quantity of an analyte present in a treatment area about the sensor, including but not limited to analytes such as of blood urea nitrogen, creatinine, fibrin, fibrinogen, immunoglobulins, deoxyribonucleic acids, ribonucleic acids, potassium, sodium, chloride, calcium, magnesium, lithium, hydronium, hydrogen phosphate, bicarbonate, and the like. However, many other analytes are also contemplated herein. Exemplary chemical/analyte sensors are disclosed in commonly owned U.S. Pat. No. 7,809,441 to Kane et al.,

Although the physiological, respiration, or chemical sensors 516 are shown as part of a medical device in FIG. 5, it is realized that in some embodiments one or more of the physiological, respiration, or chemical sensors could be physically separate from the medical device. In various embodiments, one or more of the physiological, respiration, or chemical sensors can be within another implanted medical device communicatively coupled to a medical device via telemetry interface 518. In yet other embodiments, one or more of the physiological, respiration, or chemical sensors can be external to the body and coupled to a medical device via telemetry interface 518.

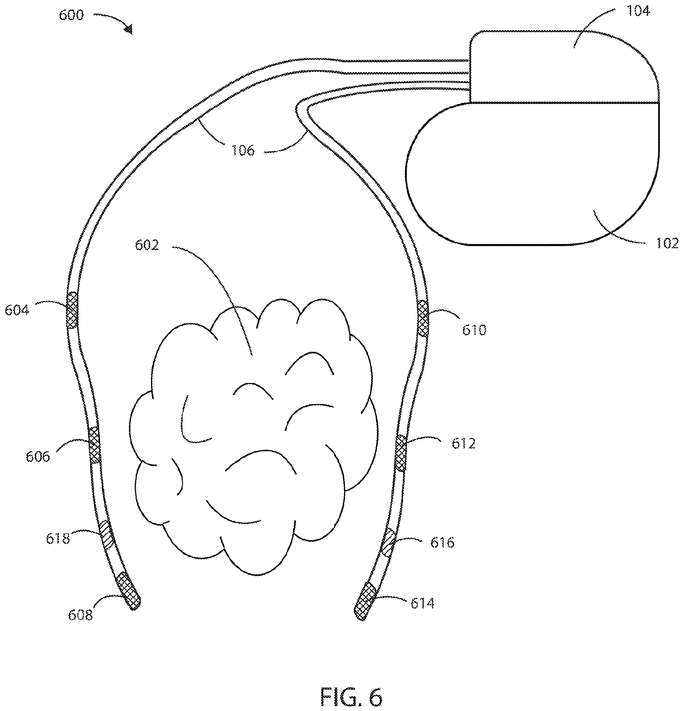

Referring now to FIG. 6, a schematic diagram of a medical device 600 is shown in accordance with the embodiments herein. Medical device 600 can include housing 102 and header 104, and one or more leads 106. Leads 106 can include one or more electrodes such as electrodes 604, 606, 608, 610, 612, or 614 disposed along the length of the leads 106. In some embodiments, electrodes 604, 606, 608, 610, 612, or 614 can include electric field generating electrodes and in other embodiments electrodes 604, 606, 608, 610, 612, or 614 can include electric field sensing electrodes. In some embodiments, leads 106 can include both electric field generating and electric field sensing electrodes.

The proximal ends of leads 106 are disposed within the header 104. The distal ends of electrical leads 106 can surround a cancerous tumor 602 such that the electrodes 604, 606, 608, 610, 612, or 614 are brought into proximity of the cancerous tumor 602. In some embodiments, the leads 106 can be positioned within the vasculature such that electrodes 604, 606, 608, 610, 612, or 614 are adjacent to or positioned within the cancerous tumor 602. However, it will be appreciated that leads 106 can be disposed in various places within or around the cancerous tumor 602. In some embodiments, the leads 106 can pass directly through the cancerous tumor 602.

In some embodiments, the leads 106 can include one or more tracking markers 616 or 618 along the length of the lead for use in determining the precise location of the electrodes relative to the tumor. In some embodiments, the one or more tracking markers can be disposed directly distal or directly proximal to the one or more electrodes disposed on the lead. In some embodiments, the tracking markers can be formed from a magnetic material. In some embodiments, the tracking markers can be formed from a radiographic material. In some embodiments, the tracking markers can be formed from a fluorographic material.

It will be appreciated that a plurality of electric field vectors can be generated between various combinations of electrodes 604, 606, 608, 610, 612, or 614 disposed along leads 106 to create an electric field. For example, one or more electric field vectors can be generated between electrodes 604 and 610. Similarly, one or more electric field vectors can be generated between electrodes 606 and 612. It will also be appreciated that one or more electric field vectors can be generated between any combination of electrodes 604, 606, 608, 610, 612, or 614. In some embodiments, one or more electric field vectors can be generated between any combination of electrodes 604, 606, 608, 610, 612, or 614 and the housing 102 of medical device 400. It will be appreciated that one or more unipolar or multipolar leads can be used in accordance with the embodiments herein. In some embodiments, a combination of unipolar and multipolar leads can be used. In other embodiments, a circular lead, clamp lead, cuff lead, paddle lead, or patch lead can be used.

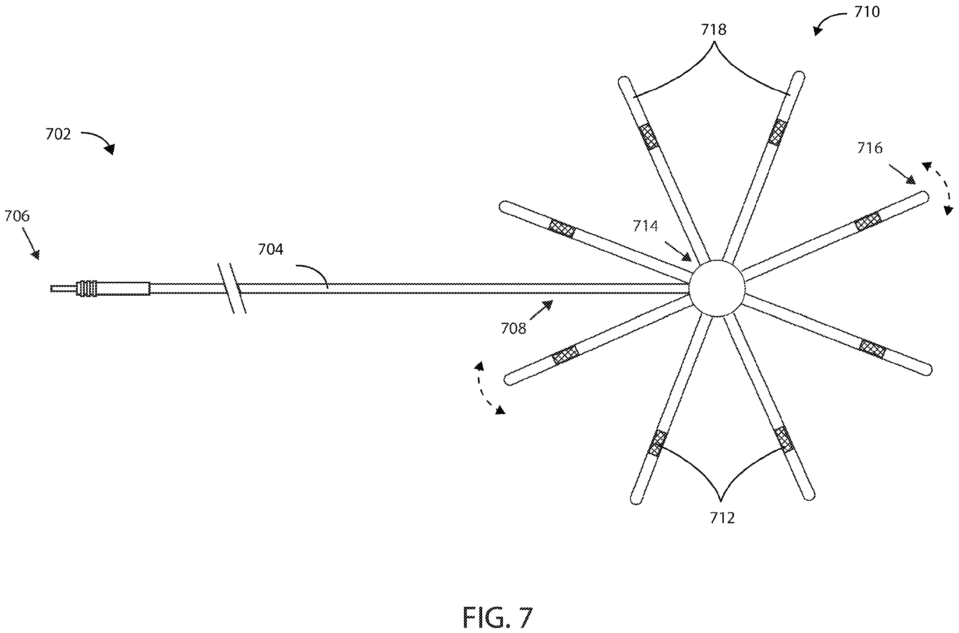

Referring now to FIG. 7, a volume-filling lead 702 is shown in accordance with the embodiments herein. Lead 702 can include a lead body 704 having a proximal end 706 and a distal end 708. The lead body 704 can define a lumen. The lead 702 can also include an expandable lead head 710 connected to the distal end 708 of the lead body 704. The expandable lead head 710 can be configured to be expanded between a first non-expanded position and a second expanded position in order to fill an intracorporeal void, such as a void that might be present after surgical resection of a cancerous tumor. The lead 702 can include two or more electrodes 712 disposed on an outer surface of the expandable lead head 710. The lead 702 can include two or more electrical conductors (not shown) configured to provide electrical communication between the two or more electrodes 712 and the proximal end 706 of the lead body 704.

In some embodiments, the expandable lead head 710 can include a proximal end 714 and a distal end 716, the expandable lead head 710 comprising one or more flexible supports 718 extending between the proximal end 714 and the distal end 716 of the expandable lead head 710. The one or more flexible supports 718 can be biased to flex outward causing the expandable lead head 710 to assume the second expanded position. In some embodiments, the one or more flexible supports 718 can include a proximal end 714 and a distal end 716, and at least one of the proximal end 714 and the distal end 716 can be configured to move relative to the lead body 704 causing the flexible supports 718 to flex outward. In some embodiments, the two or more electrodes can be disposed on or over the one or more flexible supports.

The flexible supports 718 can be formed of various materials including, but not limited to, polymers, metals, composites or the like. The size of the expandable lead head can vary. In some embodiments, the diameter of the expandable lead head is less than 2 centimeters in the first non-expanded position and greater than 2 centimeters in the second expanded position.

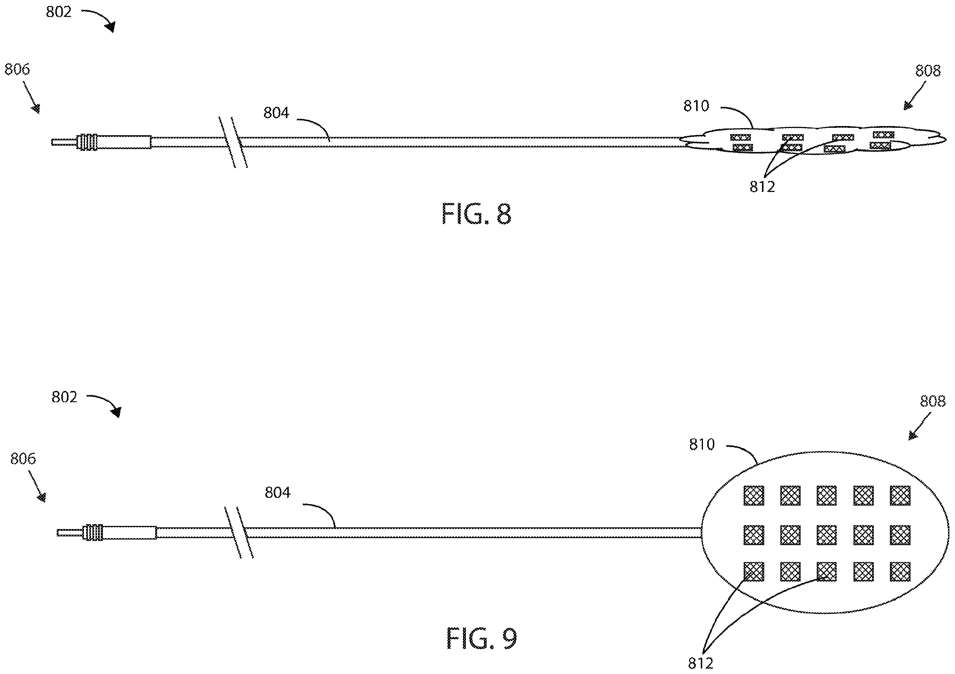

Referring now to FIG. 8, another type of volume-filling lead 802 is shown in accordance with the embodiments herein. Lead 802 can include a lead body 804 having a proximal end 806 and a distal end 808. The lead body 804 can define a lumen. The lead 802 can also include an expandable lead head 810 connected to the distal end 808 of the lead body 804. The expandable lead head 810 can be configured to be expanded between a first non-expanded position, as shown in FIG. 8, and a second expanded position, as shown in FIG. 9, in order to fill an intracorporeal void. In some embodiments, the expansion can occur passively, as in a self-expansion system, due to tension stored in the underlying lead that is released upon removal of a delivery catheter. In other embodiments, the expansion can occur via balloon expansion. The lead 802 can include two or more electrodes 812 disposed on an outer surface of the expandable lead head 810. The lead 802 can include two or more electrical conductors (not shown) configured to provide electrical communication between the two or more electrodes 812 and the proximal end 806 of the lead body 804.

Referring now to FIG. 9, volume-filling lead 802 is shown where head 810 is depicted as an expandable balloon in a second expanded position in accordance with the embodiments herein. In some embodiments, two or more electrodes 812 can be disposed on the outside of the expandable balloon. In some embodiments, two or more electrodes 812 can be disposed in an array on the outside of the expandable balloon. Each electrode 812 within the array on the outside of the expandable balloon can be sequentially activated or deactivated to provide spatial diversity for one or more electric fields about the expandable balloon. The expandable balloon can be formed from an elastomeric material. In some embodiments, the expandable balloon can be formed from an elastomeric material such as polyisobutylene (PIB) and its derivatives. In some embodiments, the expandable balloon can be formed from an elastomeric material including but not limited to polytetrafluoroethylene (ePTFE), polyethylene-co-tetrafluoroethene (ETFE), polyurethanes, silicones, poly(p-xylylene) polymers such as parylene polymers, polyether block amides such as PEBAX^{®}, nylons, or derivatives thereof. In its expanded configuration, the expandable balloon can assume a number of shapes, including a sphere, an oval, a cylinder, and the like. In some embodiments, in its second expanded position, the expandable balloon can assume the amorphous shape defined by the walls of the void into which the expandable balloon is expanded. As such, the expandable balloon can be expandable and can be compliant to fit into the void into which it is placed. In some embodiments, a lumen can be disposed within the lead body and the expandable balloon can be in fluid communication with the lumen in order to deliver a fluid to the balloon to inflate it or withdrawn a fluid from the balloon to deflate it.

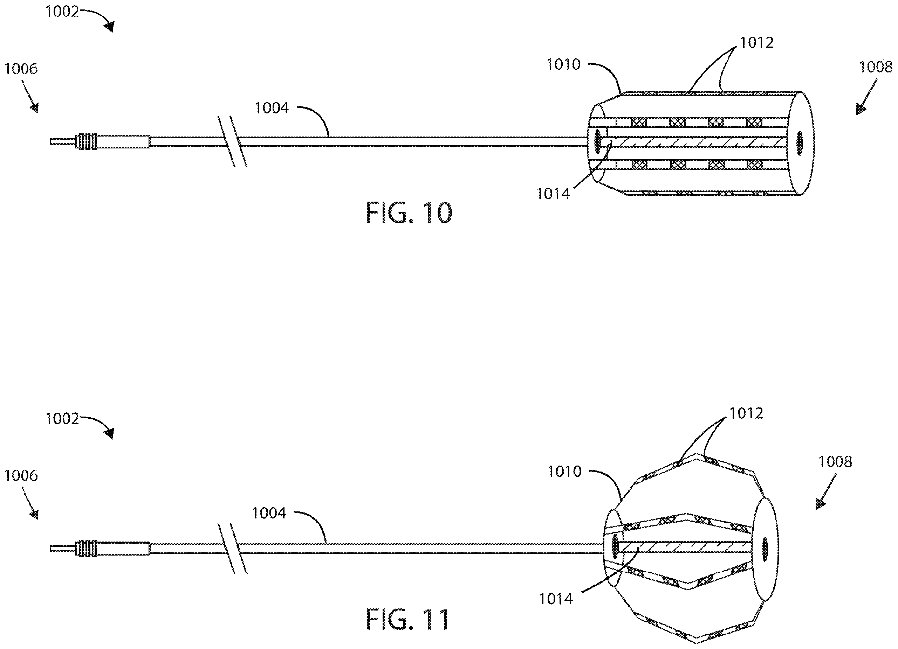

Referring now to FIG. 10, volume-filling lead 1002 is shown in accordance with the embodiments herein. Lead 1002 can include a lead body 1004 having a proximal end 1006 and a distal end 1008. The lead body 1004 can define a lumen. The lead 1002 can also include an expandable lead head 1010 connected to the distal end 1008 of the lead body 1004. The expandable lead head 1010 can be configured to be expanded between a first non-expanded position, as shown in FIG. 10, and a second expanded position, as shown in FIG. 11, in order to fill an intracorporeal void. The lead 1002 can include two or more electrodes 1012 disposed on an outer surface of the expandable lead head 1010. The lead 1002 can include two or more electrical conductors (not shown) configured to provide electrical communication between the two or more electrodes 1012 and the proximal end 1006 of the lead body 1004.

Referring now to FIG. 11, volume-filling lead 1002 is shown where expandable lead head 1010 is depicted as an expandable electrode sphere in a second expanded position in accordance with the embodiments herein. The expandable lead head 1010 can include a proximal end and a distal end. In some embodiments, the distal end of expandable lead head 1010 can move along the longitudinal axis of guide 1014 of lead 1002 in order to expand the expandable electrode sphere. In some embodiments, the proximal end of expandable lead head 1010 can move along the longitudinal axis of guide 1014 of lead 1002 to expand the expandable electrode sphere. In some embodiments, both the distal end and proximal end of expandable lead head 1010 can move along the longitudinal axis of guide 1014 of lead 1002 to expand the expandable electrode sphere. In some embodiments, two or more electrodes 1012 can be disposed on the outside of the expandable electrode sphere. In some embodiments, two or more electrodes 1012 can be disposed on the inside of the expandable electrode sphere. In some embodiments, two or more electrodes 1012 can be disposed in an array on the outside of the expandable electrode sphere. Each electrode 1012 within the array on the outside of the expandable electrode sphere can be sequentially activated or deactivated to provide spatial diversity for one or more electric fields about the expandable electrode sphere.

In some embodiments, leads 702, 802, and/or 1002 can include anywhere from 2 to 36 electrodes disposed thereon. In some embodiments, the leads 702, 802, and/or 1002 can include anywhere from 2 to 50 electrodes disposed thereon. In some embodiments, the leads 702, 802, and/or 1002 can include anywhere from 3 to 12 electrodes disposed thereon. In some embodiments, the one or more leads 702, 802, and/or 1002 can include anywhere from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 electrodes disposed thereon. It will be appreciated that the one or more leads 702, 802, and/or 1002 can include a number of electrodes falling within a range selected from the foregoing list, where any number can serve as the upper or lower bound of the range, provided that the upper bound is greater than the lower bound.

It will be appreciated that the expandable lead heads of leads 702, 802, and 1002 described herein can exist in an expanded configuration in a "free state" and yet be collapsible such that they can be implanted within a body with minimal trauma to the vasculature and surrounding areas. It will also be appreciated that necrosis due to pressure from an expandable lead head can be minimized since the expansion size of the expandable lead heads can be monitored to fit a space having a known distance. In some embodiments, where a distance is not known, the expandable lead heads can include one or more sensors to monitor the local environment to determine if pressure is too high, and the lead head can be reduced in size accordingly.

In some embodiments, the volume-filling leads 702, 802, and 1002 described above can be used in a method of treating a patient previously diagnosed with cancer. The method can include implanting a lead within a patient. The lead can include a lead body having a proximal end and a distal end, where the lead body can define a lumen. The lead can also include an expandable lead head connected to the distal end of the lead body. The lead head can be configured to be expanded between a first non-expanded position and a second expanded position in order to fill an intracorporeal void. The lead can also include two or more electrodes disposed on an outer surface of the lead head. The lead can further include two or more electrical conductors configured to provide electrical communication between the two or more electrodes and the proximal end of the lead body. The method can also include positioning the lead head within an intracorporeal void and generating one or more electric fields with the one or more electrodes.

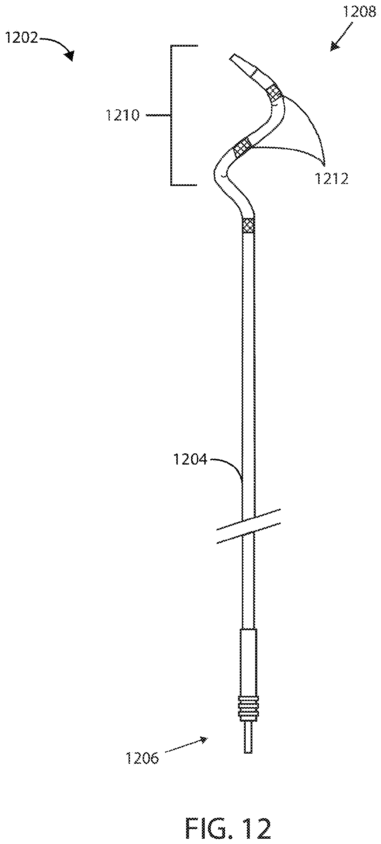

Referring now to FIG. 12, a lead 1202 for a cancer treatment system is shown in accordance with the embodiments herein. The lead 1202 can include a lead body 1204 having a proximal end 1206 and a distal end 1208, where the lead body 1204 can define a lumen. The lead 1202 can include two or more electrodes 1212 disposed on an outer surface of the distal end 1206 of the lead body 1204. The lead 1202 can also include two or more electrical conductors (not shown) configured to provide electrical communication between the two or more electrodes 1212 and the proximal end 1206 of the lead body 1204. In some embodiments, the distal end 1208 of the lead body 1204 can be in the shape of a helix 1210.

In some embodiments, the one or more electrodes 1212 are disposed on surfaces of the helix 1210 facing outward from a central axis of the helix. In some embodiments, the helix 1210 can be flexible and can decrease in diameter as pressure applied inward is increased and can increase in diameter as pressure applied inward is decreased. The helix can be sized to have various outer diameters when no pressure inward is applied (maximum diameter or full expanded diameter). In some embodiments, the helix can have an outer diameter of about 0.2, 0.5, 1.0, 1.5, 2.0, 2.5, 3.0, 3.5, 4.0, 4.5, 5.0, 6.0, 7.0, 8.0 or 10.0 centimeters, or an outer diameter falling within a range wherein any of the foregoing can serve as the upper or lower bound of the range.

In some embodiments, the lead body 1204 can include a second lumen having a substantially rigid element, such as for example a rigid wire, disposed within the second lumen, where removal of the rigid element can cause the distal end 1208 of the lead body 1204 to assume a helical shape. In other words, the rigid element can be straight and its presence within a lumen can cause the distal end 1208 of the lead body to remain substantially straight, but when it is removed then the distal end 1208 of the lead body can twist into a helical shape.

Lead 1202 can be used in a method of treating a cancerous tumor. The method can include implanting a lead within a patient. The lead 1202 can include a lead body 1204 having a proximal end 1206 and a distal end 1208, where the lead body 1204 can define a lumen. The lead 1202 can include two or more electrodes 1212 disposed on an outer surface of the distal end of the lead body 1204. The lead 1202 can also include two or more electrical conductors (not shown) configured to provide electrical communication between the two or more electrodes 1212 and the proximal end 1206 of the lead body 1204. The distal end 1208 of the lead body 1204 can be in the shape of a helix 1210. The method can also include generating one or more electric fields with the one or more electrodes 1212.

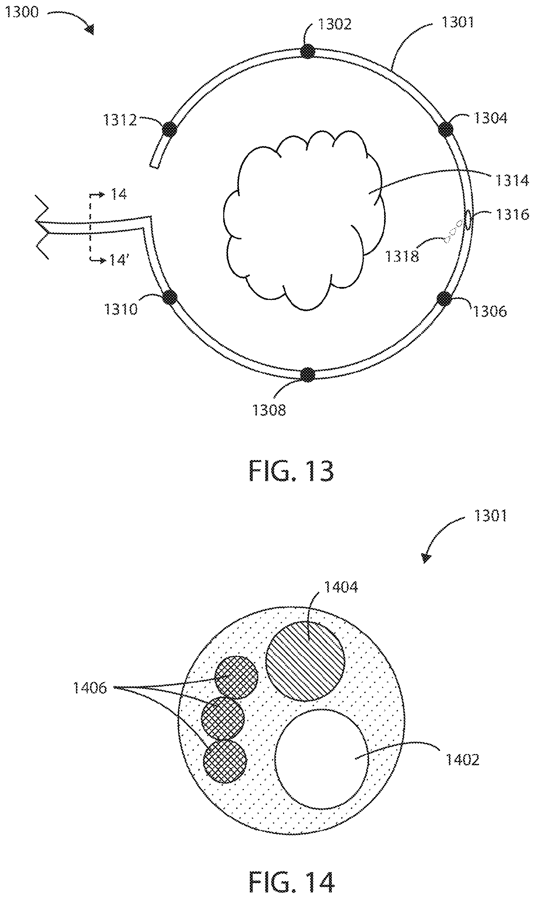

Referring now to FIG. 13, lead system 1300 surrounding a cancerous tumor 1314 is shown. Lead 1301 is a circular lead that has a semi-closed circular loop. Lead 1301 includes electrodes 1302, 1304, 1306, 1308, 1310, and 1312 disposed about its circumference. Lead 1301 can include one or more ports 1316, where port 1316 provides an outlet for a drug delivery lumen that runs the longitudinal length of the lead 1301. In some embodiments, the drug delivery lumen runs about 20, 30, 40, 50, 60, 70, 80, 90, or 100 percent of the longitudinal length of the lead 1301 or a length falling with a range, wherein any of the foregoing percents of the longitudinal length of the lead can serve as the upper or lower bound of the range.

In some embodiments, the port 1316 can be disposed between a proximal end and a distal end of the lead 1301. In some embodiments, the port 1316 can be disposed at or adjacent to a middle portion of the lead 1301, such as a middle 10, 20, 30, 40 or 50 percent of the overall length of the lead 1301. In some embodiments, the port 1316 can be disposed at or adjacent to a distal tip of the lead 1301. In some embodiments, the port 1316 can be on or in a surface of the lead 1301 along a distal half of the overall longitudinal length of the lead 1301. The drug delivery lumen can be used to deliver one or more drugs 1318 to the site of the cancerous tumor 3114 through one or more ports 1316.

Referring now to FIG. 14, a schematic cross-sectional view of lead 1301 is shown along line 14-14' of FIG. 13. Lead 1301 can include one or more components, including but not limited to drug delivery lumen 1402, guide wire lumen 1404, and conducting core wires 1406. Drug delivery lumen 1402 can deliver one or more drugs, such as steroids or chemotherapy agents, to the site of the tumor in a single bolus or periodically via a metered pump. In some embodiments, drug delivery lumen 1402 can be connected to a metered pump at or near the distal end of lead 1301.

### Leads and Electrodes

The leads described herein can be placed into the body near the site of a cancerous tumor using a number of techniques. Placement of one or more leads can include using techniques such as transvascular placement, tunneling into the subcutaneous space, and/or surgical placement. In some embodiments, the placement of one or more leads can include placement via one or more natural body orifices. The leads can be placed adjacent to or within a cancerous tumor. In some embodiments, multiple leads can be used near to or far from the cancerous tumor.

In some embodiments one or more leads described herein can be placed in the subcutaneous space. Electrodes on leads placed in the subcutaneous space can be used as the primary near-field generating electrode or as a far-field field generating electrode. In some embodiments, electrodes on leads placed in the subcutaneous space can be used as the primary near-field generating electrode or as a far-field field generating electrode in conjunction with the housing of a medical device. Likewise, one or more leads can be placed transvascularly to act as far-field field generating electrodes in conjunction with an electrode at or near the site of the cancerous tumor or in conjunction with the housing of a medical device.

The leads and electrodes described herein can include additional functional and structural features. In some embodiments, the leads can include those that are compatible with imaging and treatment techniques, including but not limited to MRI (magnetic resonance imaging), X-ray imaging, deep brain stimulation techniques, and/or radiation therapy. In some embodiments, the leads can include one or more conductor cores made from conducting materials. The conductor cores can be formed from conducting materials including metals and/or other conducting materials. Metals can include, but are not limited to, palladium, platinum, silver, gold, copper, aluminum, various alloys including stainless steel, nickel-cobalt alloys such as MP35N^{®} and the like. In some embodiments, the conductor core can be a multifilar coil, including but not limited to a bifilar coil, a trifilar coil, and a quadfilar coil.

In some embodiments, electrodes can be disposed along the length of one or more leads as described herein. Suitable materials for use in the electrodes described herein can include metals such as palladium, to minimize coupling and artifact generation in magnetic fields. In some embodiments, electrodes can be made from other metals and/or other conducting materials. Metals can include, but are not limited to, palladium, platinum, platinum alloys such as platinum-iridium alloy, gold, copper, tantalum, titanium, various alloys including stainless steel, and the like. In some embodiments, electrodes can be in the form of wound coils that can provide an added benefit of increased surface area without compromising flexibility of the electrodes. In some embodiments, the implantable device housing can serve as an electrode.

The leads described herein can also include one or more electrodes disposed along the length of the lead. The leads can include two or more electrodes disposed along the length of the lead. In some embodiments, the electrodes can be tip electrodes found at the distal end of the lead. In other embodiments, the electrodes can be ring electrodes found along the lead but not at the tip of the lead. In some embodiments, the electrodes can be coil electrodes. In some embodiments, a ring or tip electrode can be positioned in or adjacent to a tumor or cancerous tissue and a coil electrode can be positioned farther from the tumor or cancerous tissue in order to help provide spatial diversity to the generated electric fields. In some embodiments, one or more electrodes can have a length along the lengthwise axis (e.g., proximal to distal axis) of about 0.5, 1, 1.5, 2, 3, 4, 5, 7.5, 10, 15, 20, 30, 40, 50, 75, 100 mm or more. In some embodiments, one or more of the electrodes can have a length falling within a range wherein any of the foregoing distances can serve as the upper or lower bound of the range, provided that the upper bound is greater than the lower bound.

The leads can be unipolar, bipolar, or multipolar. In some embodiments, a unipolar lead can include a lead that generates an electric field between one electrode and the housing of the medical device. In some embodiments, a bipolar lead can include a lead that can generate and electric field between two electrodes disposed along the lead, or between both electrodes and the housing of the medical device. In some embodiments, a multipolar lead can include a lead that can generate an electric field between the more than two electrodes disposed along the lead, between more than two electrodes and the housing of the medical device, or any number of combinations of configurations of electrodes and the housing of the medical device.

The electrodes suitable for use here can be made of conductive polymers such as carbon filled silicone, polyacetylene, polypyrrole, polyaniline, polytiophene, polyfuran, polyisoprene, polybutadiene, polyparaphenylene, and the like. In other embodiments, the electrodes can be insulated. In some embodiments, the insulation surrounding and electrode can include microporous insulators to prevent cellular apposition, yet still allow for current flow. Microporous insulators can be made from a number of the insulating materials described herein, including but not limited to polytetrafluoroethylene (ePTFE), polyethylene-co-tetrafluoroethene (ETFE), polyurethanes, silicones, poly(p-xylylene) polymers such as Parylene polymers, polyether block amides such as PEBAX^{®}, nylons, or derivatives thereof. In some embodiments, the electrodes can be coated with various materials, including but not limited to hydrogels or fractal coatings such as iridium oxide, titanium oxide, tantalum pentoxide, other metal oxides, poly(p-xylylene) polymers such as Parylene, and the like.

A number of lead fixation techniques and configurations can be used in accordance with the embodiments herein. Some non-limiting examples of lead fixation techniques can include biocompatible glue fixation, talon fixation, helix coil fixation, passive centering of the lead in the vascular system, tine fixation within the localized vascular system, spiral bias fixation within the localized vascular system, compression fixation, suture sleeve fixation, and the like. In some examples, the leads embodied herein can be placed within the vascular system surrounding or adjacent to the site of the cancerous tumor. In other embodiments, the leads embodied herein can be place surgically at or within or surrounding the site of the cancerous tumor.

The leads suitable for use herein can also include one or more open lumens that run the entire longitudinal length of, or a select portion of the longitudinal length of the lead. In some embodiments, the open lumen can include an integrated biopsy apparatus suitable for obtaining biopsy samples from a cancerous tumor site on a periodic basis to monitor disease progression and/or regression. Leads having an open lumen can also be configured to include an integrated drug delivery lumen that can deliver one or more drugs, such as steroids or chemotherapy agents, to the site of the tumor in a single bolus or periodically via a metered pump. The leads can include one or more portals disposed along the length of the lead to provide an outlet for drug delivery at or near the site of a cancerous tumor.

In some embodiments a portion of the lead or the entire lead can include a drug eluting coating. In some embodiments, the drug eluting coating can include an anti-inflammatory agent, such as a steroid. In some embodiments, the steroid can be dexamethasone. In other embodiments, the drug eluting coating can include a chemotherapy agent. In some embodiments, the chemotherapy agent can include a taxane or derivatives thereof, including but not limited to paclitaxel, docetaxel, and the like. In other embodiments, the drug eluting coating can be configured to release additional classes of chemotherapy agents, including, but not limited to alkylating agents, plant alkaloids such as vinca alkaloids, cytotoxic antibiotics, topoisomerase inhibitors, and the like. In some embodiments, the drug eluting coating can be configured to release the drug from the coating in a time-release fashion.

The leads herein can adopt a number of shapes or configurations. In some embodiments, the leads can be linear and in other embodiments the leads can be circular. A circular lead may be a completely closed loop or it may be a semi-closed loop. In some embodiments, the lead can include a bendable core that can allow the lead to be shaped into many configurations, including but not limited to a U shape, an S shape, a spiral shape, a half circle, an oval, and the like.

In yet other examples, the leads suitable for use herein can include fluorimetric or magnetic markers that can assist the clinician in precise placement at or near the site of a cancerous tumor. The leads can also include integrated pH sensors for detecting the change in the pH at or near the cancerous tumor or other chemical sensors suitable for analyzing the concentration of a chemical analyte of interest.

### Therapy Parameters

Successful treatment of cancerous tumors can depend on a number of variables, including electric field strength, frequency, cell heterogeneity, cell size, cancer cell type, tumor size, and location within the body. A variety of therapy parameters can be implemented using the medical devices described herein. One or more therapeutic parameter sets can be programmed into the memory of the medical devices and implemented by the control circuitry 306, shown in FIG. 3. Exemplary therapeutic parameter sets can include those that implement the following concepts: sweeping through a range of frequencies; stacking of one or more frequencies simultaneously; stepping through one or more frequencies sequentially; the spatial or temporal delivery of one or more electric fields; sweeping through a range of electric field strengths; applying an effective spinning electric field; modulating a voltage control mode or a current control mode; implementing one or more duty cycles; pulse width modulation; manipulation of the waveform shape and/or pulse sequence; and the occasional use of high frequency or high electric fields strength pulses.

The therapeutic parameter sets can be programmed into a medical device to operate autonomously, or they can be queried and manipulated by the patient or a clinician using an external computation device such as a programmer, a home-based unit, and/or a mobile unit (e.g. a cellular phone, personal computer, smart phone, tablet computer, and the like). In other embodiments, the therapeutic parameter sets can be wirelessly communicated to the medical device from an external computation device. Frequencies and/or electric field strengths suitable for use in any of the therapeutic parameter sets herein are discussed above with respect to electric field generating circuit 320. In some embodiments, one or more therapeutic parameter sets can be implemented simultaneously. In other embodiments, one or more therapeutic parameter sets can be implemented in an alternating fashion.

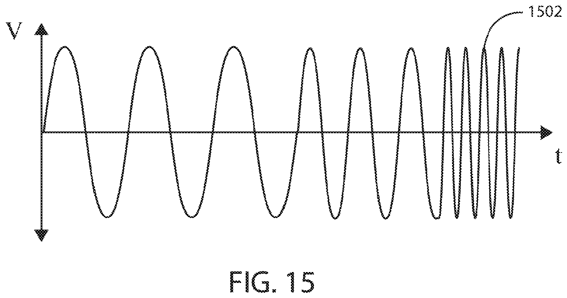

Referring now to FIG. 15, exemplary plot 1502 shows an example of sweeping through a range of frequencies at the site of a cancerous tumor. Plot 1502 shows an alternating electric field, where the frequency is increased over time as the therapy is applied to the cancerous tumor. In some embodiments, a frequency sweep can include alternating between a first frequency sweep covering a range of about 100 kHz to 300 kHz and a second frequency sweep covering a range about 200 kHz to 500 kHz. It will be appreciated that sweeping through a first and second frequency range as described can be performed indefinitely throughout the course of the therapy.

### Electric Field Generators

The medical devices embodied herein can include electric field generators particularly suited for therapeutic and diagnostic techniques used during the course of treatment for a cancerous tumor. In some embodiments, the electric field generators suitable for use herein can include those that have been treated by radiation hardening to make the components resistant to the damaging effects of radiation therapy treatments often prescribed as a main line treatment for cancerous tumors. Electric field generators can include components such as those described in reference to FIGS. 3 and 5 above.

Electric field generators embodied herein can be programmed with any number of therapeutic parameter sets as described. The electric field generators can be programmed prior to implant, or they can be programmed by a clinician using an external computation device such as a programmer, a home-based unit, and/or a mobile unit (e.g. a cellular phone, personal computer, smart phone, tablet computer, and the like). In some embodiments, therapy parameters can be delivered to the electric field generator via a telemetry circuit. In some embodiments, the electric field generator can include a recharge circuit communicatively coupled to a receiver coil to facilitate transcutaneous recharging of the medical device. In some embodiments, the electric field generator can communicate wirelessly between the receiver coil and an external charging device.

The invention is defined by claim 1. Preferred embodiments are defined in the dependent claims. Further aspects, examples and embodiments disclosed herein are for exemplary purpose only and do not form part of the invention.

## Claims

1. An implantable lead for a cancer treatment system comprising:
a lead body having a proximal end and a distal end, the lead body defining a lumen;
an expandable lead head connected to the distal end of the lead body, the lead head configured to be expanded between a first non-expanded position and a second expanded position in order to fill an intracorporeal void;
two or more electrodes disposed on an outer surface of the lead head;
two or more electrical conductors configured to provide electrical communication between the two or more electrodes and the proximal end of the lead body;
wherein the expandable lead head comprises an expandable balloon configured to assume an amorphous shape defined by the walls of the void when the expandable balloon is expanded into a second expanded position.

2. The lead of any of claims 1 and 3-8, the lead head comprising a proximal end and a distal end, the lead head comprising one or more flexible supports extending between the proximal end and the distal end.

3. The lead of any of claims 1-2 and 4-8, the one or more flexible supports biased to flex outward causing the lead head to assume the second expanded position.

4. The lead of any of claims 1-3 and 5-8, the one or more flexible supports comprising a proximal end and a distal end, wherein at least one of the proximal end and the distal end of the flexible supports are configured to move relative to the lead body causing the flexible supports to flex outward.

5. The lead of any of claims 1-4 and 6-8, wherein the diameter of the expandable lead head is less than 2 centimeters in the first non-expanded position and greater than 2 centimeters in the second expanded position.

6. The lead of any of claims 1-5 and 7-8, the two or more electrodes disposed on the one or more flexible supports.

7. The lead of any of claims 1-6 and 8, the lead head comprising an expandable balloon.

8. The lead of any of claims 1-7, wherein the two or more electrodes are disposed outside of the expandable balloon.

9. The lead of any of claims 1-8 comprising:
two or more electrodes disposed on an outer surface of a distal end of the lead body;
wherein the distal end of the lead body comprises a helix.

10. The lead of any of claims 1-9 and 11-12, wherein the one or more electrodes are disposed on surfaces of the helix facing outward from a central axis of the helix.

11. The lead of any of claims 1-10 and 12, wherein the helix is flexible and can decrease in diameter as pressure applied inward is increased and can increase in diameter as pressure applied inward is decreased.

12. The lead of any of claims 1-11, the helix having an outer diameter of at least 1 centimeter when no pressure is applied inward.

## Patentansprüche

1. Implantierbare Leitung für ein Krebstherapiesystem, die aufweist:
einen Leitungskörper mit einem proximalen Ende und einem distalen Ende, wobei der Leitungskörper ein Lumen bildet;
einen expandierbaren Leitungskopf, der mit dem distalen Ende des Leitungskörpers verbunden ist, wobei der Leitungskopf so konfiguriert ist, dass er zwischen einer ersten nicht expandierten Position und einer zweiten expandierten Position expandiert wird, um einen intrakorporalen Hohlraum zu füllen;
zwei oder mehr Elektroden, die auf einer Außenfläche des Leitungskopfs angeordnet sind;
zwei oder mehr elektrische Leiter, die so konfiguriert sind, dass sie für elektrische Kommunikation zwischen den zwei oder mehr Elektroden und dem proximalen Ende des Leitungskörpers sorgen;
wobei der expandierbare Leitungskopf einen expandierbaren Ballon aufweist, der so konfiguriert ist, dass er eine amorphe Form annimmt, die durch die Wände des Hohlraums gebildet wird, wenn der expandierbare Ballon in eine zweite expandierte Position expandiert wird.

2. Leitung nach einem der Ansprüche 1 und 3 bis 8, wobei der Leitungskopf ein proximales Ende und ein distales Ende aufweist und der Leitungskopf eine oder mehrere flexible Stützen aufweist, die sich zwischen dem proximalen Ende und dem distalen Ende erstrecken.

3. Leitung nach einem der Ansprüche 1 bis 2 und 4 bis 8, wobei die eine oder die mehreren flexiblen Stützen vorgespannt sind, um sich nach außen zu biegen, wodurch der Leitungskopf die zweite expandierte Position einnimmt.

4. Leitung nach einem der Ansprüche 1 bis 3 und 5 bis 8, wobei die eine oder die mehreren flexiblen Stützen ein proximales Ende und ein distales Ende aufweisen, wobei das proximale Ende und/oder das distale Ende der flexiblen Stützen so konfiguriert sind, dass sie sich relativ zum Leitungskörper bewegen, wodurch sich die flexiblen Stützen nach außen biegen.

5. Leitung nach einem der Ansprüche 1 bis 4 und 6 bis 8, wobei der Durchmesser des expandierbaren Leitungskopfs kleiner als 2 Zentimeter in der ersten nicht expandierten Position und größer als 2 Zentimeter in der zweiten expandierten Position ist.

6. Leitung nach einem der Ansprüche 1 bis 5 und 7 bis 8, wobei die zwei oder mehr Elektroden auf der einen oder den mehreren flexiblen Stützen angeordnet sind.

7. Leitung nach einem der Ansprüche 1 bis 6 und 8, wobei der Leitungskopf einen expandierbaren Ballon aufweist.

8. Leitung nach einem der Ansprüche 1 bis 7, wobei die zwei oder mehr Elektroden außerhalb des expandierbaren Ballons angeordnet sind.

9. Leitung nach einem der Ansprüche 1 bis 8, die aufweist:
zwei oder mehr Elektroden, die auf einer Außenfläche eines distalen Endes des Leitungskörpers angeordnet sind;
wobei das distale Ende des Leitungskörpers eine Helix aufweist.

10. Leitung nach einem der Ansprüche 1 bis 9 und 11 bis 12, wobei die eine oder die mehreren Elektroden auf Oberflächen der Helix angeordnet sind, die von einer Mittelachse der Helix nach außen weisen.

11. Leitung nach einem der Ansprüche 1 bis 10 und 12, wobei die Helix flexibel ist und ihren Durchmesser verkleinern kann, wenn nach innen ausgeübter Druck erhöht wird, und ihren Durchmesser vergrößern kann, wenn nach innen ausgeübter Druck verringert wird.

12. Leitung nach einem der Ansprüche 1 bis 11, wobei die Helix einen Außendurchmesser von mindestens 1 Zentimeter hat, wenn kein Druck nach innen ausgeübt wird.

## Revendications

1. Fil implantable pour un système de traitement du cancer comprenant :
un corps de fil ayant une extrémité proximale et une extrémité distale, le corps de fil définissant une lumière ;
une tête de fil extensible reliée à l'extrémité distale du corps de fil, la tête de fil étant conçue pour être étendue entre une première position non étendue et une seconde position étendue afin de remplir un vide intracorporel ;
deux électrodes ou plus disposées sur une surface externe de la tête de fil ;
deux conducteurs électriques ou plus conçus pour assurer une communication électrique entre les deux électrodes ou plus et l'extrémité proximale du corps de fil ;
dans lequel la tête de fil extensible comprend un ballonnet extensible conçu pour prendre une forme amorphe définie par les parois du vide lorsque le ballonnet extensible est étendu dans une seconde position étendue.

2. Fil selon l'une quelconque des revendications 1 et 3 à 8, la tête de fil comprenant une extrémité proximale et une extrémité distale, la tête de fil comprenant un ou plusieurs supports flexibles s'étendant entre l'extrémité proximale et l'extrémité distale.

3. Fil selon l'une quelconque des revendications 1 à 2 et 4 à 8, les un ou plusieurs supports flexibles étant contraints à fléchir vers l'extérieur amenant la tête de fil à prendre la seconde position étendue.

4. Fil selon l'une quelconque des revendications 1 à 3 et 5 à 8, les un ou plusieurs supports flexibles comprenant une extrémité proximale et une extrémité distale, dans lequel au moins l'une de l'extrémité proximale et de l'extrémité distale des supports flexibles est conçue pour se déplacer par rapport au corps de fil amenant les supports flexibles à fléchir vers l'extérieur.

5. Fil selon l'une quelconque des revendications 1 à 4 et 6 à 8, dans lequel le diamètre de la tête de fil extensible est inférieur à 2 centimètres dans la première position non étendue et supérieur à 2 centimètres dans la seconde position étendue.

6. Fil selon l'une quelconque des revendications 1 à 5 et 7 à 8, les deux électrodes ou plus étant disposées sur les un ou plusieurs supports flexibles.

7. Fil selon l'une quelconque des revendications 1 à 6 et 8, la tête de fil comprenant un ballonnet extensible.

8. Fil selon l'une quelconque des revendications 1 à 7, dans lequel les deux électrodes ou plus sont disposées à l'extérieur du ballonnet extensible.

9. Fil selon l'une quelconque des revendications 1 à 8, comprenant :
deux électrodes ou plus disposées sur une surface externe d'une extrémité distale du corps de fil ;
dans lequel l'extrémité distale du corps de fil comprend une hélice.

10. Fil selon l'une quelconque des revendications 1 à 9 et 11 à 12, dans lequel les une ou plusieurs électrodes sont disposées sur des surfaces de l'hélice faisant face vers l'extérieur à partir d'un axe central de l'hélice.

11. Fil selon l'une quelconque des revendications 1 à 10 et 12, dans lequel l'hélice est flexible et peut se réduire en diamètre lorsqu'une pression appliquée vers l'intérieur est augmentée et peut augmenter en diamètre lorsqu'une pression appliquée vers l'intérieur est réduite.

12. Fil selon l'une quelconque des revendications 1 à 11, l'hélice ayant un diamètre externe d'au moins 1 centimètre lorsqu'aucune pression n'est appliquée vers l'intérieur.
